# EUROPEAN PATENT APPLICATION

(11) **EP 4 785 993 A1**
(43) Date of publication of application: **05.08.2026**
(21) Application number: 26154626.1
(22) Date of filing: 28.01.2026
(51) Int. Cl.: A61N 1/375

(54) **BIOSTIMULATOR TRANSPORT SYSTEM HAVING A MOTION CONVERTER**

(30) Priority: 03.02.2025 US 202563753381 P; 27.01.2026 US 202619461602
(71) Applicant: Pacesetter, Inc., Sylmar, CA 91342 (US)
(72) Inventor: JACKSON, Aaron, Sylmar, CA 91342 (US)
(74) Representative: Barker Brettell Sweden AB

(57) **Abstract**

A biostimulator transport system (300) includes a catheter shaft (402) having a catheter lumen (502) extending longitudinally to a distal catheter end (404). The biostimulator transport system (300) includes a docking cap (320) coupled to the catheter shaft (402) and having a docking cavity (512). A drive shaft (503) extends through the catheter lumen (502) to a distal shaft end (504). The biostimulator transport system (300) includes a motion converter (506) coupling the drive shaft (503) to the docking cap (320). The motion converter (506) converts longitudinal motion of the drive shaft (503) into rotational motion of the docking cap (320).

## Description

### TECHNICAL FIELD

The present disclosure relates to biostimulators and related biostimulator systems. More specifically, the present disclosure relates to biostimulator transport systems having motion conversion mechanisms.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor. A delivery system can be used to deliver the leadless cardiac pacemakers to the target anatomy.

### SUMMARY

Existing delivery systems transmit torque to a leadless cardiac pacemaker by rotating the entire delivery system at a handle such that rotational movement is transferred through a torque shaft hypotube, a torque shaft coil, and a docking cap assembly to the leadless cardiac pacemaker. A construction of the existing delivery systems, e.g., long catheter lengths and/or several material stiffness transitions along those lengths, can result in delivery control issues, such as torque lag, torque whip, or torque loss. More particularly, direct transmission of torque from the handle to the pacemaker may be inefficient or ineffective in existing delivery systems. Accordingly, delivery systems that convert input forces at the handle to controllable torque at the pacemaker could be beneficial.

A biostimulator transport system is described. In an embodiment, the biostimulator transport system includes a catheter shaft having a catheter lumen extending longitudinally to a distal catheter end. The biostimulator transport system includes a docking cap coupled to the catheter shaft and having a docking cavity. The biostimulator transport system includes a drive shaft extending through the catheter lumen to a distal shaft end. The biostimulator transport system includes a motion converter coupling the catheter shaft to the docking cap. The motion converter converts longitudinal motion of the drive shaft into rotational motion of the docking cap.

A biostimulator system including a biostimulator mounted on the biostimulator transport system is also described. Furthermore, a method of using the biostimulator transport system to rotate a biostimulator is also described.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 illustrates a diagrammatic cross section of a patient heart illustrating an example implantation of a biostimulator in a target anatomy, in accordance with an embodiment.
FIG. 2 is a side view of a biostimulator, in accordance with an embodiment.
FIG. 3 is a perspective view of a biostimulator transport system, in accordance with an embodiment.
FIG. 4 illustrates a side view of a distal portion of a biostimulator system, in accordance with an embodiment.
FIG. 5 illustrates a section view of a distal portion of a biostimulator transport system, in accordance with an embodiment.
FIG. 6 illustrates a perspective view of a distal portion of a biostimulator transport system having a motion converter in a first state, in accordance with an embodiment.
FIG. 7 illustrates a perspective view of a distal portion of a biostimulator transport system having a motion converter in a second state, in accordance with an embodiment.
FIG. 8 illustrates a flowchart of a method of rotating a docking cap, in accordance with an embodiment.

### DETAILED DESCRIPTION

Embodiments describe a biostimulator system including a biostimulator for pacing, e.g., septal pacing. The biostimulator system may, however, be used in other applications, such as deep brain stimulation. Thus, reference to the biostimulator as being a cardiac pacemaker for pacing, or septal pacing, is not limiting.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a component of a biostimulator, a biostimulator transport system, or a biostimulator system. Similarly, "proximal" may indicate a second direction opposite to the first direction. Such terms are provided to establish relative frames of reference, however, and are not intended to limit the use or orientation of a biostimulator or a biostimulator transport system to a specific configuration described in the various embodiments below.

In an aspect, a biostimulator transport system incorporates a motion converter to convert longitudinal motion of a drive shaft to rotational motion of a docking cap. The longitudinal motion can be input to the drive shaft at a proximal end of the biostimulator transport system, e.g., via a handle. The longitudinal motion, which may be robustly transmitted to a distal end of the biostimulator transport system, can be efficiently converted into rotational motion. Accordingly, the docking cap can be controllably rotated to drive a biostimulator, e.g., via torque, into a target anatomy. The efficient transmission and conversion of motion can avoid drawbacks of existing solutions, such as torque lag, torque whip, and torque loss.

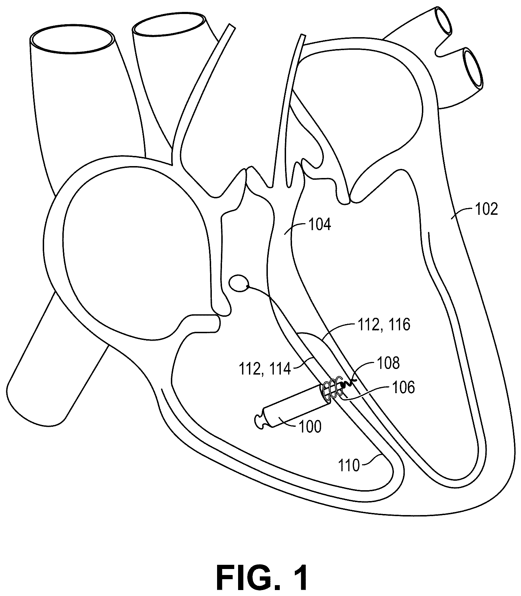

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulators 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 106, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 106 may be positioned for deep septal pacing at respective bundle branches 112 in the septum 104. For example, an active electrode of the pacing element 108 can be positioned at the left bundle branch 114 in the septum 104. Similarly, the fixation element 106 can be positioned at the right bundle branch 116 in the septum 104. Optionally, one of the elements may be at a bundle branch and the other element may not be at a bundle branch.

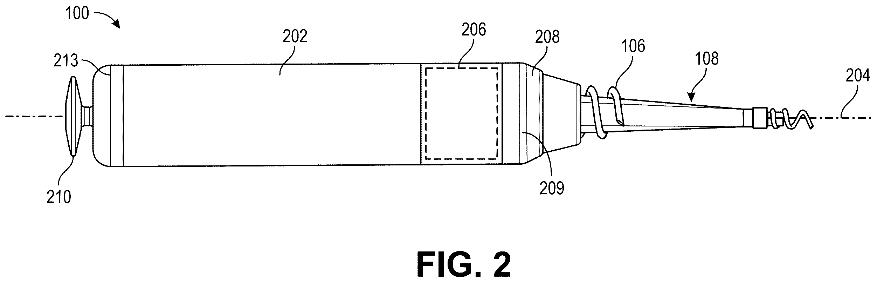

Referring to FIG. 2, a side view of a biostimulator is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of the pacing element 108 that acts as an active electrode and/or a portion of the fixation element 106 or a housing 202 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes the housing 202 having a longitudinal axis 204. The housing 202 can contain a primary battery to provide power for pacing, sensing, and communication, which may include, for example, bidirectional communication. The housing 202 can optionally contain an electronics compartment 206 (shown by hidden lines) to hold circuitry adapted for different functionality. For example, the electronics compartment 206 can contain circuits for sensing cardiac activity from the electrodes, circuits for receiving information from at least one other device via the electrodes, circuits for generating pacing pulses for delivery to tissue via the electrodes, or other circuitry. The electronics compartment 206 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 202. The fixation element 106 can include a helical fixation element and/or several tines, as described below. More particularly, the biostimulator 100 can include a header assembly having a flange 208 coupled to a distal housing end 209 of the housing 202, and the fixation element 106 can be coupled to and extend distal to the flange 208. The fixation element 106 can extend about the longitudinal axis 204. For example, in the case of a helical fixation element, the element can spiral about the longitudinal axis to a distal tip. In the case of a fixation element 106 having several tines, the tines can be arranged about the longitudinal axis and extend to respective distal tips. Accordingly, when the biostimulator 100 is delivered to the target tissue, the distal tip(s) of the fixation element 106 can pierce the tissue and the housing 202 can be rotated or pushed to affix the fixation element 106 to the target tissue.

In an embodiment, the biostimulator 100 includes an attachment feature 210. The attachment feature 210 can be mounted on a proximal housing end 213 of the housing 202. More particularly, the attachment feature 210 can be mounted on an opposite end of the housing 202 from the fixation element 106 and the pacing element 108, which as described above, can be coupled to the distal housing end 209 of the housing 202. The attachment feature 210 can facilitate precise delivery or retrieval of the biostimulator 100. For example, the attachment feature 210 can be formed from a rigid material to allow a biostimulator transport system to engage, e.g., via a docking cap, the attachment feature 210 and transmit torque and/or axial loads to the attachment feature 210 to plunge and rotate the fixation element 106 or the pacing element 108 into the target tissue.

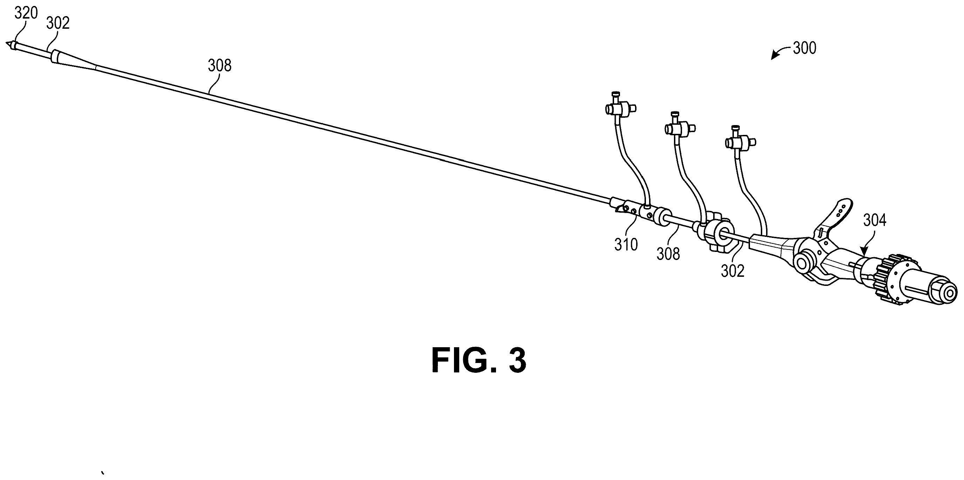

Referring to FIG. 3, a perspective view of a biostimulator transport system is shown in accordance with an embodiment. Leadless pacemakers or other leadless biostimulators 100 can be delivered to and retrieved from a patient anatomy using a biostimulator transport system 300. In some implementations, the biostimulator transport system 300 is a delivery system for delivering the leadless pacemaker to the target tissue. In some implementations, the biostimulator transport system 300 is a retrieval system for retrieving the leadless pacemaker from the target tissue. As described below, the biostimulator transport system 300 can include a docking cap to transmit rotational motion to the biostimulator 100, e.g., by transmitting torque to the attachment feature 210 received within the docking cap, to screw into the target anatomy.

The biostimulator transport system 300 can include an elongated catheter 302 extending distally from a handle 304 to a distal catheter end. The elongated catheter 302 can be a deflectable catheter, and an operator can use the handle 304 to steer a distal catheter end in the patient. In an embodiment, the biostimulator transport system 300 includes a guide catheter 308 mounted on the elongated catheter 302. The guide catheter 308 can be slidably disposed on the elongated catheter 302 such that a distal portion of the guide catheter 308 can slide distally over the distal catheter end of the elongated catheter 302 and/or the biostimulator 100, which may be mounted on the distal catheter end (not shown). Similarly, the biostimulator transport system 300 can include an introducer hub assembly 310 mounted on the guide catheter 308. The introducer hub assembly 310 can be slidably disposed on the guide catheter 308 such that a distal portion of the introducer hub assembly 310 can slide distally over the distal catheter end of the elongated catheter 302 and/or the distal portion of the guide catheter 308. More particularly, the introducer hub assembly 310 can be inserted into an access sheath to gain access to the patient vasculature, and after access is established, the distal portion of the guide catheter 308 and/or the distal catheter end of the elongated catheter 302 can be advanced through the access sheath into the patient.

The distal catheter end of the elongated catheter 302 may be selectively connectable to the biostimulator 100. More particularly, the biostimulator 100 can be mounted on the distal catheter end of the elongated catheter 302. The biostimulator may be held at the distal catheter end by a retention mechanism. For example, the biostimulator transport system 300 may include a snare, several tethers, a threaded shaft, etc. that can engage and releasably hold the attachment feature 210 of the biostimulator 100. The retention mechanism can release the attachment feature 210 to implant the biostimulator 100 at a target site. The biostimulator 100 can be protected by a protective sheath of the distal portion of the guide catheter 308 during delivery and/or retrieval of the biostimulator 100 from the patient. Accordingly, the biostimulator 100 can be advanced into the patient along with the distal catheter end.

The leadless pacemaker system can be used to implant one or more biostimulators 100 within an atrium and/or a ventricle of a heart 104 of the patient. Implantation of each biostimulator 100 may be achieved, in part, by endocardial insertion of the biostimulators 100. For example, the elongated catheter 302 of the leadless pacemaker system can include a drive shaft, within an outer member of the elongated catheter 302, coupled to a docking cap 320. The docking cap 320 can have a docking cavity to receive the attachment feature 210 of the biostimulator 100. The drive shaft can be moved longitudinally, e.g., advanced or retracted, to transmit torque to and rotate the docking cap 320, as described below. The rotating docking cap 320 may impart rotation to the attachment feature 210. Accordingly, movement can be transmitted through the drive shaft and, as described below, a motion converter, to rotate the biostimulator 100 in a first direction, e.g., clockwise. Rotating the biostimulator 100 when the fixation element 106 is in contact with the heart tissue can cause the fixation element 106 to screw into the heart tissue and affix the biostimulator 100 to the heart tissue. Similarly, removal and retrieval of the biostimulators 100 may be accomplished endocardially. For example, the drive shaft of the elongated catheter 302 can be moved longitudinally, e.g., retracted, to impart rotation to the attachment feature 210 in a second direction, e.g., counterclockwise. The rotation can transmit torque through the docking cap 320 to the attachment feature 220 to disengage the biostimulator 100 from the heart tissue. The motion converter is described in more detail below.

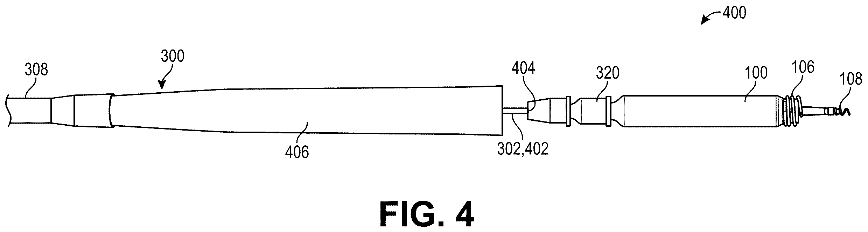

Referring to FIG. 4, a side view of a distal portion of a biostimulator system is shown in accordance with an embodiment. A biostimulator system 400 includes the biostimulator 100 coupled to the biostimulator transport system 300. For example, the biostimulator 100 can be mounted on the docking cap 320 of the biostimulator transport system 300. The docking cap 320 can be coupled to the elongated catheter 302. More particularly, the docking cap 320 can be coupled to a catheter shaft 402 of the elongated catheter 302. The catheter shaft 402 can be an outer member of the elongated catheter 302, which extends longitudinally along the longitudinal axis 204 to a distal catheter end 404. The docking cap 320 can be mounted on or otherwise connected to the distal catheter end 404. In an embodiment, and as described below, the docking cap 320 is rotatable relative to the catheter shaft 402.

In an embodiment, the biostimulator transport system 300 includes the guide catheter 308 that can be advanced or retracted to cover or expose the biostimulator 100. For example, in the illustrated state, a protective sleeve 406 of the guide catheter 308 can be retracted to expose the biostimulator 100 and the docking cap 320 to a surrounding environment. The protective sleeve 406 can include a flexible tubular element having a flared end to allow the sleeve to be advanced smoothly over the docking cap 320. When the guide catheter 308 is advanced, the protective sleeve 406 can slide over the docking cap 320 and the biostimulator 100 to cover the fixation element 106 and/or pacing element 108 of the biostimulator 100. In the advanced state, the protective sleeve 406 may therefore protect the biostimulator 100 from snagging on tissue. Accordingly, the biostimulator system 400 can be moved through a vasculature of a patient in the advanced state. By contrast, when the protective sleeve 406 is in the retracted state, the biostimulator 100 can engage the target anatomy and be rotated by the docking cap 320 to screw into or out of the target tissue, as described below.

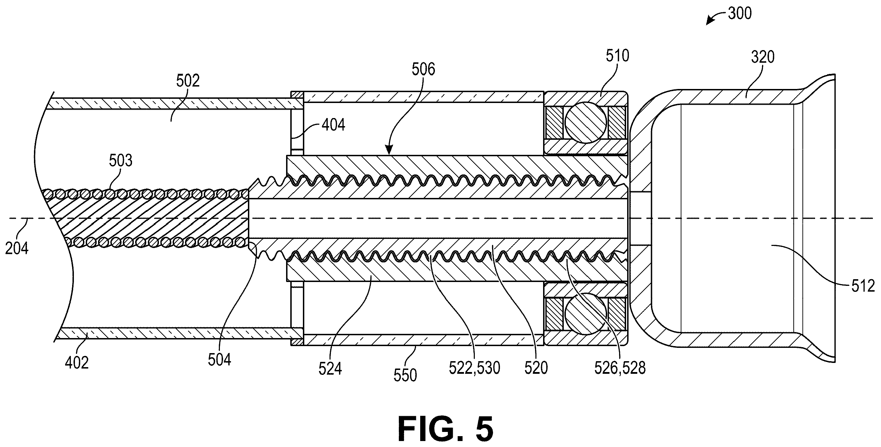

Referring to FIG. 5, a section view of a distal portion of a biostimulator transport system is shown in accordance with an embodiment. The catheter shaft 402 of the biostimulator transport system 300 can include a catheter lumen 502 extending longitudinally, e.g., along the longitudinal axis 204, to the distal catheter end 404. The catheter lumen 502 can contain power or motion transmission components used to transmit motion from the handle 304 to the docking cap 320. For example, a drive shaft 503 can extend through the catheter lumen 502 to a distal shaft end 504, which may be coupled to a motion converter 506. More particularly, the motion converter 506 can couple, e.g., interconnect, the drive shaft 503 to the docking cap 320. Accordingly, movement of the drive shaft 503 may be converted into corresponding movement of the docking cap 320.

The docking cap 320 can rotate relative to the catheter shaft 402. For example, the biostimulator transport system 300 can include a bearing 510 rotationally coupling the docking cap 320 to the catheter shaft 402. In an embodiment, the bearing 510 includes a ball bearing 510 having inner and outer races that move relative to each other based on an intermediate roller connection. The races can be respectively coupled to the catheter shaft 402 or the docking cap 320. For example, an inner race can be rotationally fixed relative to the docking cap 320 and an outer race can be rotationally fixed relative to the catheter shaft 402. The races, however, may rotate about the longitudinal axis 204 relative to each other by rolling over balls of the bearing 510 and, thus, the docking cap 320 can rotate relative to the catheter shaft 402.

The docking cap 320 may rotate to drive the biostimulator 100 into or out of the target tissue. In an embodiment, the docking cap 320 has a docking cavity 512 sized to receive the attachment feature 210 of the biostimulator 100. When the attachment feature 210 is placed in the docking cavity 512, a mating surface, e.g., a drive tooth, of the docking cap 320 can engage the attachment feature 210. Accordingly, rotation of the docking cap 320 can cause the tooth to transmit torque to the attachment feature 210 and the housing 202 of the biostimulator 100. The biostimulator 100 may therefore be driven into or out of the target tissue.

In an embodiment, the motion converter 506 converts longitudinal motion of the drive shaft 503 into rotational motion of the docking cap 320. The motion converter 506 can include a linear-to-rotational torque mechanism, which has a lead screw structure that can be actuated by a cable structure to advance or retract and drive rotation of a nut structure. For example, the motion converter 506 can include a screw shaft 520 having a shaft thread 522 to provide the lead screw structure. The nut structure can be provided by a screw nut 524 having a nut thread 526. The shaft thread 522 can engage and mesh with the nut thread 526. Furthermore, the engaging threads can have a pitch that allows for linear motion of the shaft thread 522 along the longitudinal axis 204 to cause the nut thread 526 to rotate about the longitudinal axis 204.

The drive shaft 503 can be pushed or pulled to advance or retract the screw shaft 520 through an internal channel of the screw nut 524. More particularly, the screw nut 524 can have a tubular shape such that the nut thread 526 is an internal thread 528 surrounding the internal channel, and the screw shaft 520 can move through the internal channel and have an external thread 530 on an outer surface engaging the nut thread 526. The linear motion of the screw shaft 520 can cause the external thread 530 to press against and rotate the internal thread 528. Furthermore, the screw nut 524 can be attached to the bearing 510. For example, an outer surface of the screw nut 524 can be welded or otherwise bonded to an inner race of the bearing 510, and may be directly or indirectly fixed to the docking cap 320. Accordingly, pushing or pulling the drive shaft 503 to translate the screw shaft 520 within the screw nut 524 can cause the docking cap 320 to rotate.

An outer race of the bearing 510 can be directly or indirectly coupled to the catheter shaft 402. For example, the outer race may, optionally, be connected to a clutch 550, which may be connected to the catheter shaft 402. As described below, the clutch 550 can allow the docking cap 320 to dynamically connect to the motion converter 506. The clutch 550 can operatively couple the bearing 510 the catheter shaft 402 such that the outer race is fixed relative to the catheter shaft 402. In any case, when the inner race is operatively coupled to the docking cap 320 and the inner race rotates, the docking cap 320 can rotate relative to the catheter shaft 402. Torque may thereby be applied through the docking cap 320 to the attachment feature 210 of the biostimulator 100 to cause rotation of the biostimulator 100 relative to the catheter shaft 402.

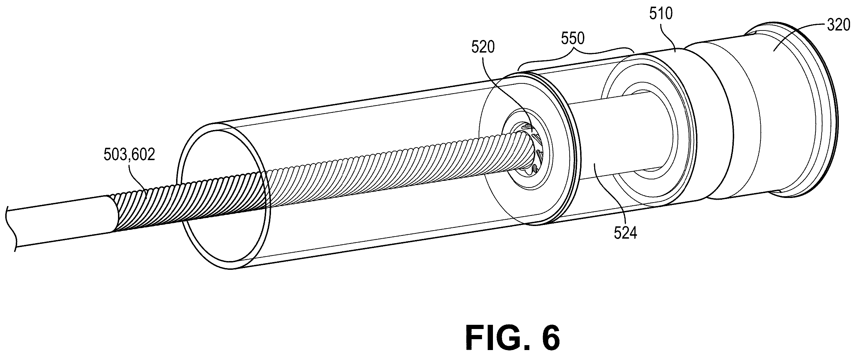

Referring to FIG. 6, a perspective view of a distal portion of a biostimulator transport system having a motion converter in a first state is shown in accordance with an embodiment. The materials of the linear-to-rotational torque mechanism can be tailored to the intended function. In an embodiment, the drive shaft 503 can be longitudinally rigid and laterally flexible. For example, the torque shaft may be formed from a cable 602 that can bend laterally relative to the longitudinal axis 204. Axial elongation of the cable 602 may be limited, however. In an embodiment, the cable 602 is braided to increase axial stiffness. Alternatively, the cable 602 may be a spiral cut tube having uncut segments that limit axial elongation. In any case, the drive shaft 503 can be flexible in bending to facilitate tracking through tortuous anatomy, however, the shaft may be axially stiff to ensure good force transmission through the shaft when pushing or pulling to actuate the lead screw.

Similar to the drive shaft 503, the docking transmission may be formed from flexible components. In an embodiment, one or more of the screw shaft 520 or the screw nut 524 are formed from a polymeric material. For example, the screw shaft 520 and/or the screw nut 524 may be formed from an elastomeric material. The elastomeric material can be injection molded to form the shaft or the nut components. The elastomer can be flexible and, thus, a bending stiffness of the docking transmission may be low. The components may engage primarily through longitudinal motion, however, and the docking transmission may therefore convert translational motion into rotational motion.

Flexibility of a distal portion of the biostimulator transport system 300 may also be influenced by a length of the docking transmission, or the docking transmission components. More particularly, it will be appreciated that the overlap of the screw shaft 520 and the screw nut 524 can increase stiffness and, to reduce overall stiffness, a length of either the shaft or the nut may be minimized. For example, the shaft thread 522 may be shorter than the nut thread 526 to enhance system flexibility. The thread length may be measured longitudinally or along a spiral length of the thread and, in either case, the shorter shaft thread 522 can result in a longitudinal length of the screw shaft 520 being less than a longitudinal length of the screw nut 524. For example, the length of the screw nut 524 may be one third or less of the length of the nut thread 526. Accordingly, an interface between the threads may be shortened, which can reduce component overlap and increase overall flexibility of the docking transmission and system.

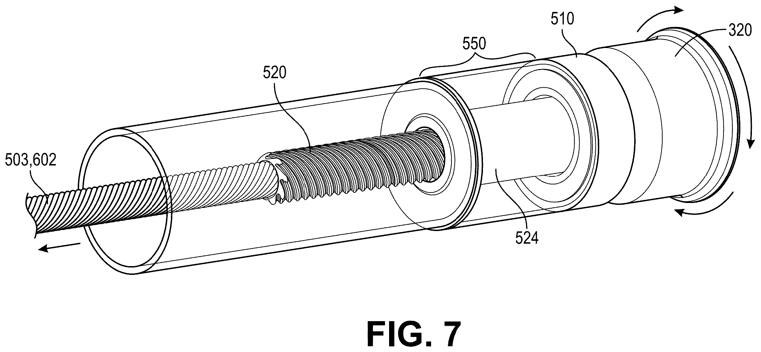

Referring to FIG. 7, a perspective view of a distal portion of a biostimulator transport system having a motion converter in a second state is shown in accordance with an embodiment. Actuation of the cable 602 may be via a push or pull action input at the handle 304. For example, the handle 304 can include an input element, such as a knob or button, which may be pressed forward and backward on the handle 304. The input element can connect to the drive shaft 503 to convey the actuation force through the shaft to the screw shaft 520. More particularly, moving the input element can actuate the cable 602 to cause the screw nut 524 to rotate via the threaded connection to the translating drive shaft 503.

In an embodiment, as shown in FIG. 7, pulling back on the drive shaft can cause rotation of the docking cap. When the lead screw is pulled back, the screw nut 524 can spin a predetermined amount per distance of travel. More particularly, a pitch of the shaft thread 522 can determine a rotational range of the docking cap 320 for a given retraction distance. For example, the threads of the screw shaft 520 and screw nut 524 may be M2 (2 mm) threads having a travel distance per turn of 3 mm. A total rotational distance of the docking cap 320 may be achieved by pulling or pushing the lead screw through a corresponding distance. For example, the drive shaft 503 can be pulled to retract the screw shaft 520 9 mm, resulting in three full rotations of the docking cap 320 (e.g., a 1080° rotation).

In addition to controlling rotational distance through thread selection, controllability of the docking transmission can be affected by thread choice. For example, the thread pitch may be selected to cause more docking cap rotation per drive shaft translation (coarse control) or less rotation per translation (fine control). Similarly, handedness of the threads may be changed to determine rotation in a first direction or a second direction. More particularly, the threads may be right-handed, as shown, to produce clockwise rotation in response to shaft retraction, or left-handed to produce counterclockwise rotation in response to shaft retraction. Such handedness selection may be used to utilize a thread direction that is useful for a delivery system or retrieval system. For example, the right-handed thread may be useful in a delivery system used for delivering a biostimulator 100 having a right-handed helix, and the left-handed thread may be useful in a retrieval system used for retrieving such biostimulator 100.

In certain situations, a user may need to rotate the docking cap 320 more than is permitted by a single throw of the drive shaft 503. For example, the screw nut 524 may cause three rotations of the docking cap 320 when the drive shaft 503 is pulled proximally by a maximum stroke length, however, the implantation process may require more than three rotations to adequately engage the target tissue with the fixation element 106. Accordingly, the biostimulator transport system 300 can optionally include the clutch 550 to allow the screw shaft 520 to be reset. More particularly, the screw shaft 520 can be moved back to a starting position, causing counter-rotation of the screw nut 524, without causing a corresponding counter-rotation of the docking cap 320. Resetting the motion converter 506 allows the user to perform multiple throws of the input element and to generate correspondingly more rotations of the docking cap 320.

In an embodiment, the clutch 550 has an engaged state in which the docking cap 320 is rotationally fixed relative to the motion converter 506, and a disengaged state in which the docking cap 320 is rotationally free relative to the motion converter 506. For example, the clutch mechanism can decouple the docking cap 320 from the screw nut 524 to allow the motion converter 506 to be actuated without conveying torque from the screw nut 524 to the docking cap 320. More particularly, when the screw nut 524 is decoupled from the docking cap 320, the screw shaft 520 can be moved, e.g., pushed forward, through the central channel of the screw nut 524 to cause the screw nut 524 to rotate from a final position to a start position without transmitting such rotation to the docking cap 320. The clutch 550 may then be placed in the engaged state to reconnect the screw nut 524 to the docking cap 320 and to allow additional docking cap actuations. For example, the screw shaft 520 can once again be moved proximally through the screw nut 524 to generate additional rotations of the docking cap 320.

The clutch 550 can include various clutch mechanisms. For example, the clutch 550 may include a set of engaging dogs/slots or meshing teeth between the docking cap 320 and the screw nut 524. When the teeth are engaged, e.g., by pulling the docking cap 320 against the screw nut 524, rotation of the screw nut 524 can be transmitted to the docking cap 320. By contrast, when the teeth are disengaged, e.g., by allowing the docking cap 320 to float forward relative to the screw nut 524, the rotation of the screw nut 524 may not be transmitted to the docking cap 320 and the docking transmission can be reset from the final position to the starting position.

In an alternative embodiment, engagement/disengagement of the clutch 550 can be performed by engaging or disengaging the screw nut 524 from the bearing 510, which may intervene between the docking cap 320 and the motion converter 506. For example, the screw nut 524 can engage and disengage a surface of the inner race, which may in turn connect to the docking cap 320, to permit transfer of rotation. When the clutch 550 is disengaged, the screw shaft 520 can be reset without causing rotation of the screw nut 524 to rotate the docking cap 320.

It will be appreciated that additional options for allowing multiple throws of the screw shaft 520 through the screw nut 524 may be selected. In an embodiment, the biostimulator 100 is undocked from the docking cap 320 to allow the screw shaft 520 to be reset to the start location. The docking cap 320 may then be pulled back into the docking cap 320 to allow a second throw of the screw shaft 520 to drive the screw nut 524. The screw nut 524 can rotate the docking cap 320 and advance the biostimulator 100 into the target tissue. Resetting and actuating the docking transmission may be performed any number of times to achieve the desired docking cap 320 rotation amount.

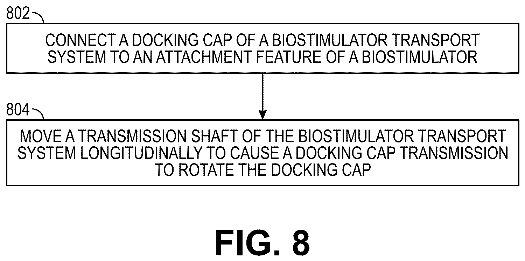

Referring to FIG. 8, a flowchart of a method of rotating a docking cap is shown in accordance with an embodiment. Rotation of the docking cap 320 may be performed, for example, when delivering the biostimulator 100 into target tissue or retrieving the biostimulator 100 from the target tissue. At operation 802, the docking cap 320 of the biostimulator transport system 300 is connected to the attachment feature 210 of the biostimulator 100. For example, the attachment feature 210 can be loaded onto one or more tethers prior to delivery to a target anatomy or may be captured by a snare during retrieval from the target anatomy. The attachment feature 210 may then be pulled into and received by the docking cavity 512. At operation 804, the drive shaft 503 of the biostimulator transport system 300 is moved longitudinally to cause the motion converter 506 to rotate the docking cap 320. For example, a user can move the input element on the handle 304 to drive rotation of the biostimulator 100 when screwing the fixation element 106 into or out of the target tissue. Accordingly, the motion converter 506 may operate to convert linear movement of the drive shaft 503 into rotational movement of the docking cap 320 during biostimulator 100 delivery or retrieval. The docking cap 320 can rotate, causing the biostimulator 100 to screw into or out of the target tissue.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator transport system (300), comprising:
a catheter shaft (402) having a catheter lumen (502) extending longitudinally to a distal catheter end (404);
a docking cap (320) coupled to the catheter shaft (402) and having a docking cavity (512);
a drive shaft (503) extending through the catheter lumen (502) to a distal shaft end (504); and
a motion converter (506) coupling the drive shaft (503) to the docking cap (320), wherein the motion converter (506) is configured to convert longitudinal motion of the drive shaft (503) into rotational motion of the docking cap (320).

2. The biostimulator transport system of claim 1, wherein the motion converter (506) includes a screw shaft (520) having a shaft thread (522), and a screw nut (524) having a nut thread (526), and wherein the shaft thread (522) is configured to mesh with the nut thread (526).

3. The biostimulator transport system of claim 2, wherein one or more of the screw shaft (520) or the screw nut (524) are formed from a polymeric material.

4. The biostimulator transport system of claim 2 or 3, wherein the shaft thread (522) is an external thread and the nut thread (526) is an internal thread.

5. The biostimulator transport system of any one of claims 2 to 4, wherein the shaft thread (522) is shorter than the nut thread (526).

6. The biostimulator transport system of any one of claims 1 to 5, wherein the drive shaft (503) is longitudinally rigid and laterally flexible.

7. The biostimulator transport system of any one of claims 1 to 6, further comprising a bearing (510) rotationally coupling the docking cap (320) to the catheter shaft (402).

8. The biostimulator transport system of claim 7, wherein the motion converter (506) includes a screw nut (524) attached to the bearing (510).

9. The biostimulator transport system of claims 2 and 8, wherein the screw shaft (520) is configured to move longitudinally through an internal channel of the screw nut (524) to cause the screw nut (524) to rotate.

10. The biostimulator transport system of any one of claims 1 to 9, further comprising a clutch (550) having an engaged state in which the docking cap (320) is rotationally fixed relative to the motion converter (506).

11. The biostimulator transport system of claim 10, wherein the clutch (550) has a disengaged state in which the docking cap (320) is rotationally free relative to the motion converter (506).

12. The biostimulator transport system of claim 10 or 11, wherein the motion converter (506) includes a screw nut (524), and wherein the clutch (550) includes meshing teeth between the docking cap (320) and the screw nut (524).

13. A biostimulator system (400), comprising: the biostimulator transport system (300) of any one of claims 1 to 12; and a biostimulator (100) mounted on the docking cap (320) of the biostimulator transport system (300).

14. The biostimulator system of claim 13, wherein the docking cavity (512) is configured to receive an attachment feature (210) of the biostimulator (100).

15. The biostimulator system of claim 14, wherein the biostimulator transport system (300) includes a snare to engage the attachment feature (210).
